# EUROPEAN PATENT APPLICATION

(11) **EP 2 810 634 A1**
(43) Date of publication of application: **10.12.2014**
(21) Application number: 13743870.1
(22) Date of filing: 31.01.2013
(51) Int. Cl.: A61G 7/05, A47C 21/08

(54) **IMMOBILISATION DEVICE FOR PATIENTS CONFINED TO BED**

(30) Priority: 31.01.2012 ES 201230100 U
(71) Applicant: Servicio Andaluz de Salud, 41071 Sevilla (ES)
(72) Inventor: GÁLVEZ RODRÍGUEZ, María Dolores, E-41071 Sevilla (ES); ROJAS RAMÍREZ, Antonio Bernabé, E-41071 Sevilla (ES)
(74) Representative: Illescas, Manuel
(86) International application number: PCT/ES2013/070050
(87) International publication number: WO 2013/113968

(57) **Abstract**

Immobilization device for bedridden patients comprising a truss with front zip, and a pair of elastic securing bands which make it possible to fix the truss to the bed headboard.

## Description

### OBJECT OF THE INVENTION

The invention is included within the field of medicine, and more specifically relates to a truss designed to immobilize patients who must remain bedridden for a long time in a certain position and sitting up to a variable degree as prescribed.

### BACKGROUND OF THE INVENTION

In hospitalization units, observation areas of hospitals, patients are frequently admitted with the need for prolonged bed rest, and on many occasions it is necessary to maintain a certain inclination posture by prescription, and avoid sliding on the bed, unsuitable postures and falls of patients.

Fixing devices currently exist which are especially designed for this purpose, such as, for example the abdominal securing bands of the brand Clipbelt or bed fasteners from the brand Vettel. These fasteners fundamentally consist of a type of belt which fastens to the patient's abdominal area, and which has a pair of side bands which are fixed to the sides of the bed or side rails if any.

However, although these devices suitably resolve the problem in certain situations, they have various drawbacks. Firstly, it is not compatible with certain inclinations of the patient's bed, which tends to slide downward as the bed's inclination increases with respect to the horizontal. Furthermore, it cannot be used in the cases where it is not recommendable to compress the patient's rib cage or pelvis.

### DESCRIPTION OF THE INVENTION

The present invention discloses a novel immobilization device for bedridden patients which overcomes the aforementioned drawbacks thanks to two main characteristics: it uses a truss to secure the patient and he/she is secured to the headboard instead of to one of the sides. This device has, in first place, the advantage of not compressing the abdominal area of the patient, since the traction is performed from the sides of the truss and in a direction parallel to the patient's trunk. Furthermore, as the patient is secured to the bed headboard instead of to the sides, it avoids the possibility that he/she falls down the bed when it is inclined. This allows the patient to adopt a suitable position for non-invasive mechanical ventilation, avoiding postures that increase the risk of aspiration, inadequate ventilation and, therefore, also the risk of lack of ventilation. The prescription of different degrees of inclination is also necessary in patients with traumatic brain injury with increase in intracranial pressure among other indications.

The immobilization device for bedridden patients of the present invention is fundamentally formed by a truss whereto are fixed two elastic securing bands to the bed headboard. Each one of these elements is described in more detail below.

The truss, when placed on the patient, goes from the perineum to the abdomen, and has a front zip which allows access to the patient's genital area to check catheters and wash the patient. The truss can, in principle, be made from any material typical of this type of garments.

On the other hand, each elastic securing band has a first end fixed to the side area of the truss and a second end which is adapted to be fixed to the bed headboard. In principle, the elastic bands have sufficient length to go from the patient's hip area to the bed headboard. For example, this length could be between 80 cm and 140 cm, more preferably between 100 cm and 120 cm.

The fixing between the first end of the elastic bands and the sides of the truss can be performed in various ways. For example, the first end of the bands may be sewn to the sides of the truss, or said first end and the truss may have complementary Velcro pieces suitably located to perform their joining and adaptability. However, in a preferred embodiment of the invention, the sides of the truss comprise different rings whereto the elastic bands are fixed by means of knots, buttons, Velcro or others.

Thus, to use the immobilization device of the present invention it is sufficient to place the truss on the patient and fix the second ends of the elastic bands to the bed headboard beside the patient's head. The elastic bands run along the entire patient's body side area, helping stabilize his/her position, and as they are fixed to the bed headboard, they suitably secure the patient, even when the bed is inclined. Additionally, the elastic securing bands preferably comprise padded parts to improve the lateral stability throughout the patient's trunk. Furthermore, the securing through the truss avoids compressing the patient's abdominal area.

According to a preferred embodiment of the invention, the second end of the elastic bands has Velcro areas to be fixed to said bed headboard, thus allowing a fast and simple installation. Furthermore, this second end of the bands may further comprise different pads adapted to be placed on the sides of the patient's neck and head area. Thus, the second end of the bands can be crossed over the patient's head, passing over the bed headboard, suitably placing the pads to also immobilize the head and neck, something indispensable for patients with reduced consciousness, loss of muscular tone or neck injuries. These pads may have an ergonomic form complementary to the shape of the side of the patient's neck and head area.

### BRIEF DESCRIPTION OF THE FIGURES

Fig.1 shows a view of a device according to the present invention.
Fig. 2 shows a patient on which an immobilization device according to the invention has been used.

### PREFERRED EMBODIMENT OF THE INVENTION

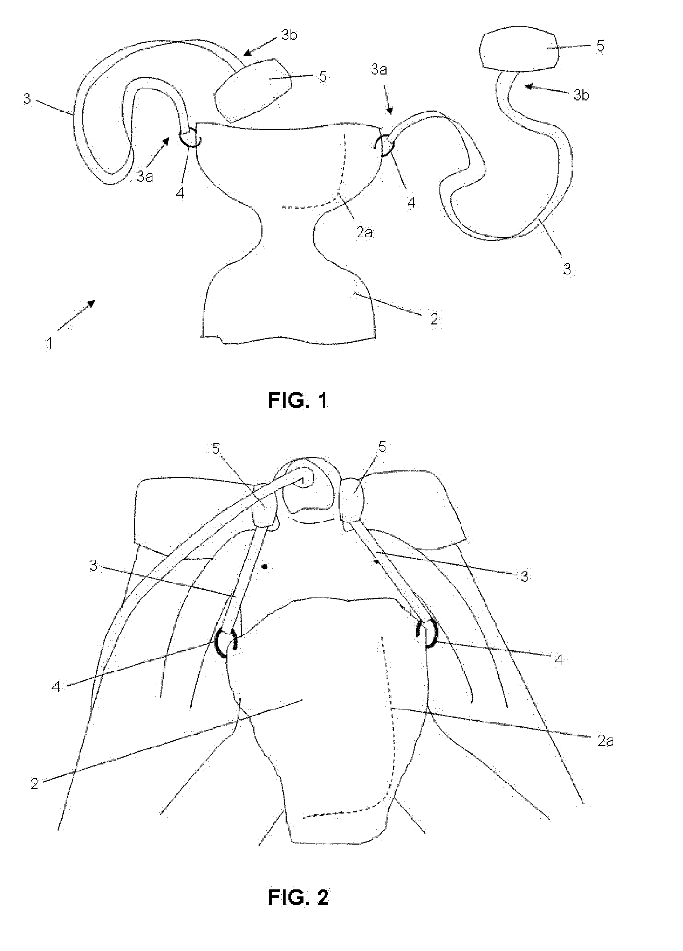

An example of device (1) according to the present invention is described below, making references to attached Figs. 1 and 2. Therein it is observed how the device (1) is formed by a large truss (2) which passes under the patient's legs and reaches the area of the abdomen. The truss (2) has a zip (2a) which serves to facilitate access to the genital area to the medical professionals for washing and checking catheters.

The sides of the truss (2) have, in this example, a ring (4) fixed whereto is fixed in turn the first end (3a) of the lateral elastic bands (3). These bands (3) have an approximate length of 100 cm to allow the fixing of its second end (3b) to the area of the headboard of the patient's bed. The fixing to the headboard can be carried out in several ways, although in one embodiment of the invention (not shown) the second end (3b) of the bands (3) has surfaces equipped with Velcro. Furthermore, the device (1) shown in this example has pads (5) fixed to the second end (3b) of the bands (3) which can be placed on the sides of the patient's head with the aim of keeping it in a suitable position in the event of reduction in level of consciousness, loss of muscular tone or coma. To do this, it would be sufficient to cross the bands (3) behind the bed headboard and place the pads (5) so that they secure the head and/or the neck of the patient. Additionally, the bands (3) may have padded areas to be more easily moulded to the patient's trunk and help keep it in a suitable position.

This novel device (1) resolves the aforementioned problems, making it possible to incline the patient's bed as much as necessary in accordance with the needs and prescription at any given time without the fear that he/she will slide downwards on the bed. At the same time it enables the stability of the trunk, head and neck.

## Claims

1. Immobilization device (1) for bedridden patients **characterized in that** it comprises:
a truss (2) which, when it is placed on the patient, goes from the perineum to the abdomen, and which has a front zip (2a) which allows access to the genital area of the patient; and
a pair of elastic securing bands (3), each one of which has a first end (3a) fixed to the side area of the truss (2) and a second end (3b) adapted to be fixed to the bed headboard.

2. Immobilization device (1) according to claim 1, where the second end (3b) of the elastic bands (3) has Velcro areas to be fixed to said bed headboard.

3. Immobilization device (1) according to any of the preceding claims, where the sides of the truss (2) comprise different rings (4) to allow the fixing of the first ends (3a) of the elastic bands (3).

4. Immobilization device (1) according to any of the preceding claims, where the elastic securing bands (3) comprise padded parts to improve the lateral stability throughout the patient's trunk.

5. Immobilization device (1) according to any of the preceding claims, where the second ends (3b) of the elastic securing bands (3) further comprise different pads (5) adapted to be placed on the sides of the patient's neck and head area.

6. Immobilization device (1) according to preceding claim, where the pads (5) have an ergonomic form complementary to the shape of the side of the patient's neck and head area.

7. Immobilization device (1) according to any of the preceding claims, where the stretched elastic securing bands (3) have a length of between 80 cm and 140 cm.

8. Immobilization device (1) according to the preceding claim, where the stretched elastic securing bands (3) have a length of between 100 cm and 120 cm.
